# EUROPEAN PATENT SPECIFICATION

(11) **EP 1 411 967 B1**
(45) Date of publication and mention of the grant of the patent: **10.08.2011**
(21) Application number: 02756467.3
(22) Date of filing: 26.06.2002
(51) Int. Cl.: A61K 38/00, C07K 14/47

(54) **BLOCKING PEPTIDE FOR INFLAMMATORY CELL SECRETION**
BLOCKIERENDES PEPTID FÜR DIE SEKRETION VON ENTZÜNDUNGSZELLEN
PEPTIDE BLOQUANT AGISSANT SUR LA SECRETION CELLULAIRE INFLAMMATOIRE

(30) Priority: 26.06.2001 US 300933 P
(43) Date of publication of application: 28.04.2004
(73) Proprietor: NORTH CAROLINA STATE UNIVERSITY, Raleigh, NC 27695-8210 (US)
(72) Inventor: MARTIN, Linda, D., Apex, NC 27502 (US); ADLER, Kenneth, B., Raleigh, NC 27613 (US); LI, Yuehua, Raleigh, NC 27606 (US)
(74) Representative: Ward, Siobhan
(86) International application number: PCT/US2002/022270
(87) International publication number: WO 2003/000027

(56) References cited:
- WO-A2-00/50062
- ADLER K B ET AL: "MYRISTOYLATED ALANINE-RICH C-KINASE SUBSTRATE PROTEIN A MAJOR INTRACELLULAR REGULATORY MOLECULE CONTROLLING SECRETION OF MUCIN BYHUMAN AIRWAY GOBLET CELLS" CHEST, THE COLLEGE, CHICAGO, IL, US, vol. 117, no. 5 Suppl 1, May 2000 (2000-05), pages 266S-267S, XP000946039 ISSN: 0012-3692
- ZHAO Y. ET AL.: 'Role of MARCKS in regulating endothelial cell proliferation' AM. J. PHYSIOL. CELL PHYSIOL. vol. 279, no. 5, November 2000, pages C1611 - C1620, XP002959324
- LI Y. ET AL.: 'MARCKS protein is a key molecule regulating mucin secretion by human airway epithelial cells in vitro' JOURNAL OF BIOLOGICAL CHEMISTRY vol. 276, no. 44, 02 November 2001, pages 40982 - 40990, XP002953475
- ELZAGALLAAI A. ET AL.: 'Platelet secretion induced by phorbol esters stimulation is mediated through phosphorylation of MARCKS: a MARCKS-derived peptide blocks MARCKS and serotonin release without affecting pleckstrin phosphorylation' BLOOD vol. 95, no. 3, 01 February 2000, pages 894 - 902, XP002959325
- ROGERS D.R.: 'Airway goblet cell hyperplasia in asthma: hypersecretory and anti-inflammatory' CLIN. EXP. ALLERGY vol. 32, no. 8, August 2002, pages 1124 - 1127, XP002959326

## Description

### Field of the Invention

The present invention relates to a MANS peptide for use in treating arthritis.

### Background of the Invention

Hypersecretion of mucus contributes to the pathogenesis of a large number of airway inflammatory diseases in both human and non-human animals. Increased mucus secretion is seen in chronic disease states such as asthma, COPD and chronic bronchitis; in genetic diseases such as cystic fibrosis; in allergic conditions (atopy, allergic inflammation); in bronchiectasis; and in a number of acute, infectious respiratory illnesses such as pneumonia, rhinitis, influenza or the common cold. Accordingly, new methods and therapeutic compounds able to decrease or lessen mucus secretion are desirable.

Accompanying hypersecretion of mucus in many of these respiratory diseases is the constant presence of inflammatory cells in the airways. These cells contribute greatly to the pathology of these diseases via the tissue damage done by the inflammatory mediators released from these cells. One example of such destruction via this chronic inflammation occurs in cystic fibrosis patients where mediators released from neutrophils (i.e. myeloperoxidase) induce the desquamation of the airway epithelial tissue.

Under-secretion of mucus also has harmful effects. Airway mucus acts as a physical barrier against biologically active inhaled particles, and may help prevent bacterial colonization of the airways and inactivate cytotoxic products released from leukocytes. King et al., Respir. Physiol. 62:47-59 (1985); Vishwanath and Ramphal, Infect. Immun, 45:197 (1984); Cross et al., Lancet 1:1328 (1984). In the eye, mucus maintains the tear film, and is important for eye health and comfort. Mucus secretion in the gastrointestinal tract also has a cytoprotective function. The role of mucus as a chemical, biological and mechanical barrier means that abnormally low mucus secretion by mucous membranes is undesirable.

Mammalian airways are lined by a thin layer of mucus produced and secreted by airway epithelial (goblet) cells and submucosal glands. In diseases such as asthma, chronic bronchitis, and cystic fibrosis, hypersecretion of mucus is a common lesion. Excess mucus can contribute to obstruction, susceptibility to infection, and even to destruction of airway walls and contiguous tissues. The major components of mucus are mucin glycoproteins synthesized by secretory cells and stored within cytoplasmic membrane-bound granules. Mucins are a family of glycoproteins secreted by the epithelial cells including those at the respiratory, gastrointestinal and female reproductive tracts. Mucins are responsible for the viscoelastic properties of mucus and at least eight mucin genes are known. Thornton, et al., J Biol. Chem. 272, 9561-9566 (1997). Mucociliary impairment caused by mucin hypersecretion and/or mucus cell hyperplasia leads to airway mucus plugging that promotes chronic infection, airflow obstruction and sometimes death. Many airway diseases such chronic bronchitis, chronic obstructive pulmonary disease, bronchiectacis, asthma, cystic fibrosis and bacterial infections are characterized by mucin overproduction. E. Prescott, et al., Eur, Respir. J., 8:1333-1338 (1995); K. C. Kim, et al., Eur. Respir. J., 10: 1438 (1997); D. Steiger, et al. Am. J Respir, Cell Mol. Biol., 12:307-314 (1995). Upon appropriate stimulation, mucin granules are released via an exocytotic process in which the granules translocate to the cell periphery where the granule membranes fuse with the plasma membrane, allowing for luminal secretion of the contents.

Despite the obvious pathophysiological importance of this process, intracellular signaling mechanisms linking stimulation at the cell surface to mucin granule release previously has only recently been elucidated. *See,* Li et al., Journal of Biological Chemistry, 276: 40982-40990 (2001). It is known that a wide variety of agents and inflammatory/humoral mediators provoke mucin secretion. These include cholinergic agonists, lipid mediators, oxidants, cytokines, neuropeptides, ATP and UTP, bacterial products, neutrophil elastase, and inhaled pollutants. See, Adler et al., Res. Immunol. 149, 245-248 (1998). Interestingly, many of these mucin secretagogues are also known to activate several protein kinases, and studies examining the regulation of excess secretion of mucin by airway epithelial cells from various species have consistently implicated involvement of either protein kinase C (PKC) or cGMP- dependent protein kinase (PKG) in the secretory process. See, *e.g.,* Ko et al., Am. J. Respir. Cell Mol. Biol. 16, 194-198 (1997); Abdullah et al., Am. J. Physiol. 273, L201-L210 (1997); Abdullah et al., Biochem. J. 316, 943-951 (1996); Larivee et al. Am, J. Respir. Cell Mol. Biol. 11, 199-205 (1994); and Fischer et al., Am. J. Respir. Cell Mol. Biol. 20, 413-422 (1999). Coordinated interactions or "cross-talk" between these two protein kinases in regulation of mucin secretion has only recently been demonstrated to involve the MARCKS proteins. *See,* Li et al., Journal of Biological Chemistry, 276: 40982-40990 (2001). However, signaling events downstream of the coordinated action of these protein kinases that ultimately leads to the exocytotic release of mucin granules have not been fully elucidated. Interestingly, similar experimentation examining release of inflammatory mediators from neutrophils suggests a similar pathway of kinase "cross-talk" regulates secretion in these inflammatory cells; thus suggesting the potential universality of secretory mechanisms that involve multiple kinases, in particular PKC and PKG.

Previously, procedures to culture normal human bronchial epithelial (NHI3E) cells in an air/liquid interface system in which the cells differentiate to a heterogeneous population containing secretory (goblet), ciliated, and basal cells that mimic their *in vivo* appearance and function was reported. Krunkosky et al., Am. J. Respir. Cell Mol. Biol. 22, 685-692 (2000). These cell cultures may provide an *in vitro* model system to study mechanisms regulating mucin secretion from human airway epithelium. Yet, there is a need in the field to understand the mechanisms regulating mucin secretion from human airway epithelium cells and to develop methods of regulating mucin secretion to improve upon anti-inflammatory therapy.

Further efforts to elucidate mechanisms responsible for secretion of inflammatory mediators from inflammatory cells may also lead to the ability to inhibit both types of secretion (mucus and inflammatory mediators) via targeting an intracellular molecule or event common to both types of secretory pathways.

### Summary of the Invention

The invention relates to a new use for the 24 amino acid, myristoylated polypeptide, also known as the MANS peptide.

Specifically, the present invention relates to a MANS peptide having the amino acid sequence of SEQ ID No 1, or an active fragment thereof, for use in treating arthritis. Also provided is the MANS peptide or an active fragment thereof, for use in treating arthritis wherein said arthritis is osteoarthritis or rheumatoid arthritis. As used herein, an "active fragment" of a MARCKS protein is one that affects (inhibits or enhances) the MARCKS protein- mediated release.

Herein the administration is generally selected from the group consisting of topical administration, parenteral administration, rectal administration, pulmonary administration, inhalation and nasal or oral administration, wherein pulmonary administration generally includes either an aerosol, a dry powder inhaler, a metered dose inhaler, or a nebulizer.

As used herein "reducing" generally means a lessening of the effects of inflammation. Preferably, inflammatory mediators are inhibited or blocked by the methods disclosed. Additionally, both the inflammation and mucus secretion may be reduced simultaneously. The term simultaneously means that both inflammation and mucus secretion are reduced at the same time.

The term "inhibiting" means a reduction in the amount of mucus secretion.

### Brief Description of the Drawings

FIGs, 1A-1D are bar graphs illustrating mucin hypersecretion by NHBE cells is maximized by activation of both PKC and PKG.
FIGs. 2A-2B demonstrate that the MARCKS protein is a key component of the mucin secretory pathway.
FIGs. 3A-3C depicts a gel illustrating that an antisense oligonucleotide directed against MARCKS down-regulates MARCKS expression and attenuates mucin hypersecretion.
FIGs 4A-4B illustrate that PKC-dependent phosphorylation releases MARCKS from the plasma membrane to the cytoplasm.
FIGSs. 5A-5C show that PKG induces dephosphorylation of MARCKS by activating PP2A.
FIG. 6 depicts bar graphs that demonstrate that PP2A is an essentialcomponent of the mucin secretory pathway.
FIG. 7 is a gel that illustrates that MARCKS associates with actin and myosin in the cytoplasm.
FIG. 8 depicts a signaling mechanism controlling mucin secretion by human airway epithelial cells.
FIG. 9 is a bar graph depicting the ability of MANS peptide to block secretion of myloperoxidase from isolated canine neutrophils,
FIG. 10 is a bar graph depicting the ability of MANS peptide to block secretion of myloperoxidase from isolated human neutrophils.
FIG. 11 is a bar graph showing that PMA stimulates a small increase in MPO secretion from LPS-stimulated human neutrophils which is enhanced in a concentration-dependent manner by co-stimulation with 8-Br-cGMP.
FIG. 12 is a bar graph showing that 8-Br-cGMP simulation has little effect on MPO secretion from LPS-stimulated human neutrophils until a co- stimulation with PMA occurs in a concentration-dependent manner.
FIG. 13 is a bar graph showing that PMA stimulates a small increase in MPO secretion from LPS-stimulated canine neutrophils which is enhanced in a concentration-dependent manner by co-stimulation with 8-13r-cGMP.
FIG. 14 is a bar graph showing that 8-Br-cGMP simulation has little effect on MPO secretion from LPS-stimulated canine neutrophils until a co- stimulation with PMA occurs in a concentration-dependent manner.
FIG. 15 is a bar graph showing that costimulation with PMA + 8-Br-cGMP is required for maximal MPO secretion from LPS-stimulated canine neutrophils.

### Detailed Description of the Preferred Embodiments

The present invention will now be described more fully hereinafter with reference to the accompanying figures, in which preferred embodiments of the invention are illustrated. This invention may, however, be embodied in different forms and should not be construed as limited to the embodiments set forth herein. Rather, these embodiments are provided so that this disclosure will be thorough and complete, and will fully convey the scope of the invention to those skilled in the art.

Unless otherwise defmed, all technical and scientific terms used herein have the same meaning as commonly understood by one of ordinary skill in the art to which this invention belongs.

The invention includes within its scope use of a pharmaceutical composition. The pharmaceutical composition comprises a therapeutically effective amount of a known compound and a pharmaceutically acceptable carrier. A "therapeutically effective" amount as used herein is an amount of a compound that is sufficient to ameliorate symptoms exhibited by a subject. The therapeutically effective amount will vary with the age and physical condition of the patient, the severity of the condition of the patient being treated, the duration of the treatment, the nature of any concurrent treatment, the pharmaceutically acceptable carrier used and like factors within the knowledge and expertise of those skilled in the art. Pharmaceutically acceptable carriers are preferably solid dosage forms such as tablets or capsules. Liquid preparations for oral administration also may be used and may be prepared in the form of syrups or suspensions, e.g., solutions containing an active ingredient, sugar, and a mixture of ethanol, water, glycerol, and propylene glycol. If desired, such liquid preparations may include one or more of following: coloring agents, flavoring agents, and saccharin. Additionally, thickening agents such as carboxymethylcellulose also may be used as well as other acceptable carriers, the selection of which are known in the art.

As stated above, the present invention relates to methods for regulating cellular secretory processes, especially those releasing inflammatory mediators from inflammatory cells. As used herein, the term "regulating" means blocking, inhibiting, decreasing, reducing, increasing, enhancing or stimulating. A number of cellular secretory processes involve the release of contents from membrane-bound vesicles. Some of the contents of these vesicles, such as those contained in inflammatory cells, have been found to be responsible for a variety of pathologies in numerous mammalian tissues. Some of the effects of these secretions appear to include damage of previously healthy tissue during inflammation. This invention provides a means of blocking secretion from any membrane-bound vesicle, including those found in inflammatory cells, by targeting a specific molecule important in the intracellular secretory pathway with a synthetic peptide. This approach may be of therapeutic importance for the treatment of a wide variety of hypersecretory and inflammatory conditions in humans and animals.

One benefit of the present invention is that it may combine a therapy that includes the direct blocking of mucus secretion with a unique anti-inflammatory therapy. A benefit of the present invention over current anti-inflammation therapies that affect a general suppression of the immune system is that the peptide is thought to block secretion of only membrane-bound components secreted from inflammatory cells. Thus, many aspects of the immune system should still function without the release of a number of damaging agents.

The compounds of the invention may regulate, *i.e*. block, inflammatory mediator release from cells. This inhibition of inflammatory production is an attractive means for preventing and treating a variety of disorders, *e.g*., diseases and pathological conditions involving inflammation. These encompass chronic inflammatory diseases including, but not limited to, osteoarthritis, multiple sclerosis, Guillain-Barre syndrome, Crohn's disease, ulcerative colitis, psoriasis, graft versus host disease, systemic lupus erythematosus and insulin-dependent diabetes mellitus. It is envisaged that the compounds described herein can also be used to treat other disorders associated with the activity of elevated levels of proinflammatory enzymes such as responses to various infectious agents and a number of diseases of autoimmunity such as rheumatoid arthritis, toxic shock syndrome, diabetes and inflammatory bowel diseases.

The peptide as described herein may also be used to combat inflammation along with therapies that will combine the anti-inflammatory activity of the peptide with its ability to block mucus secretion. Diseases that may be treated by the peptide's ability to block both inflammation and mucus secretion include but are not limited to inflammatory bowel diseases, digestive disorders *(i.e*., inflamed gall bladder, Menetier's disease) and inflammatory airway diseases. The peptide may also be used to block release of excess insulin from pancreatic islet cells.

Other proinflammatory mediators have been correlated with a variety of disease states that correlate with influx of neutrophils into sites of inflammation or injury. Blocking antibodies have been demonstrated as useful therapies against the neutrophil-associated tissue injury in acute inflammation (Harade et al, 1996, Molecule Medicine Today 2, 482). Other cells that may release inflammatory mediators include basophils, eosinophils, leukocytes, monocytes and lymphocytes, and therapies may be directed against secretion from these cells.

Although inventors do not wish to be bound to any particular theory of the invention, it is thought that the mechanisms regulating such basal secretion are different than those regulating stimulated secretion. Alternatively, basal secretory mechanisms may require less MARCKS protein than stimulated secretion. Since therapies to block MARCKS-mediated secretion are unlikely to eliminate all MARCKS function, basal secretion may accordingly be preserved.

As used herein, the term "MARCKS nucleotide sequence" refers to any nucleotide sequence derived from a gene encoding a MARCKS protein, including, for example, DNA or RNA sequence, DNA sequence of the gene, any transcribed RNA sequence, RNA sequence of the pre-mRNA or mRNA transcript, and DNA or RNA bound to protein.

Precise delivery of the MARCKS-blocking peptide may also overcome any potential limitations of blocking important secretory processes. Delivering such agents to the respiratory tract should be readily accomplished with inhaled formulations. Since these agents may be useful in treating inflammatory bowel disease, one can envision delivery of the blocking agents into the rectum/colon/intestinal tract via enema or suppositories. Injections or transdermal delivery into inflamed joints may yield relief to patients with arthritic or autoimmune diseases by limiting the secretion from localized inflammatory cells. Injection into areas surrounding nerve endings may inhibit secretion of some types of neurotransmitters, blocking transmission of severe pain or uncontrolled muscle spasms. Delivery of the peptide for the treatment of inflammatory skin diseases should be readily accomplished using various topical formulations known in the art.

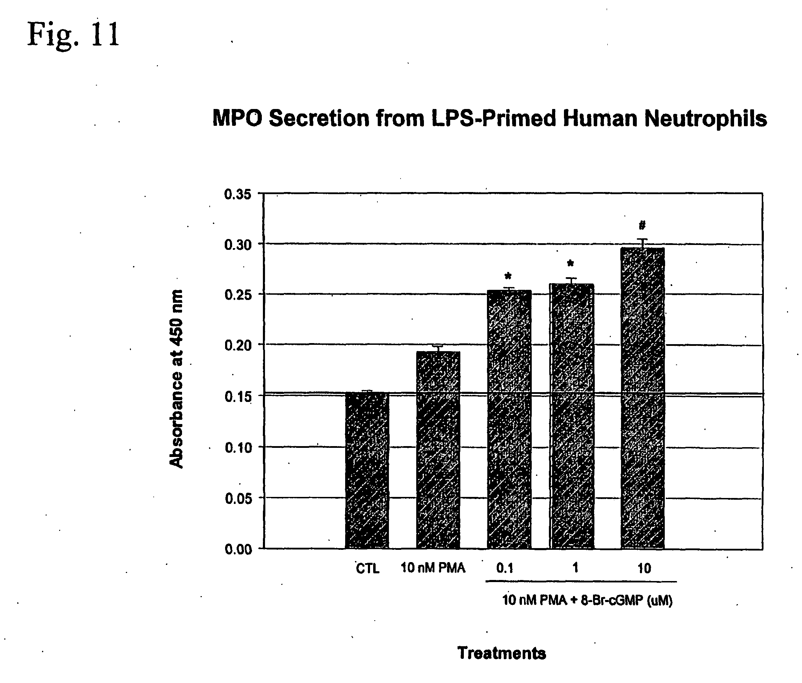

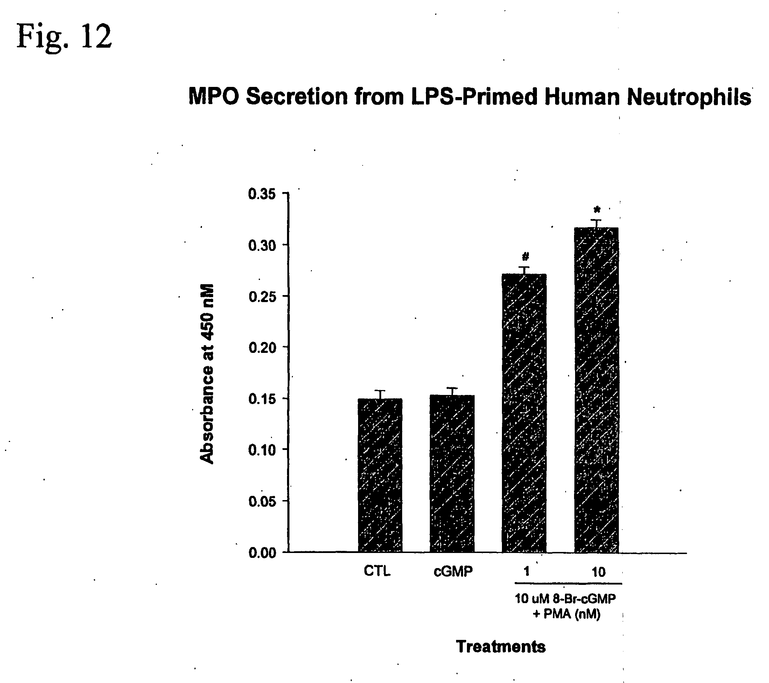

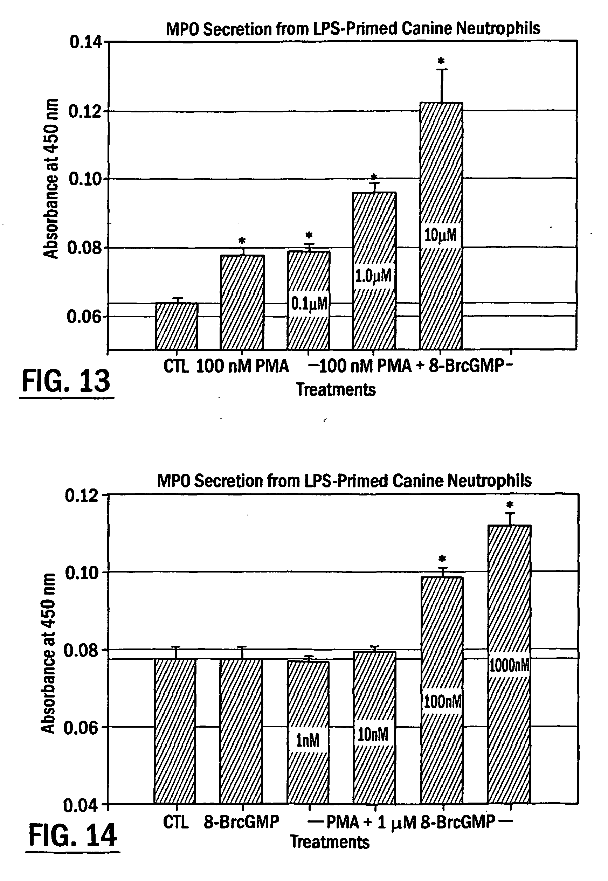

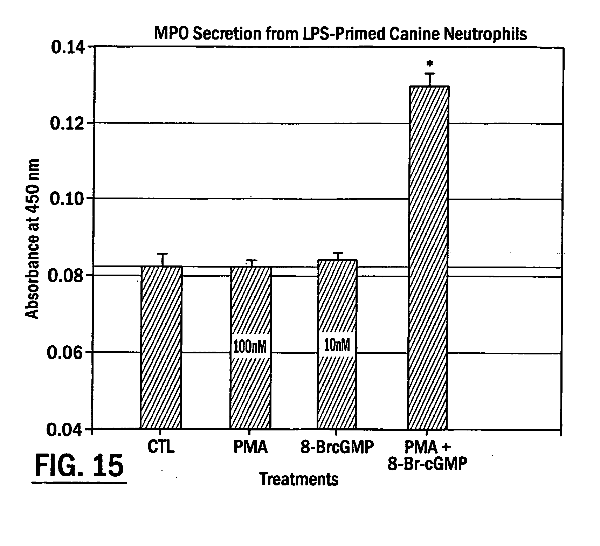

The present disclosure demonstrates that the myristoylated alanine-rich C kinase substrate (MARCKS), a widely distributed PKC substrate may be a key regulatory molecule mediating mucin granule release by normal human bronchial epithelial (NHBE) cells. Secretion of mucin from these cells may be maximized by activation of both PKC and PKG. It is believed that MARCKS serves as the point of convergence for coordinating the actions of these two protein kinases to control mucin granule release. The mechanism appears to I nvolve PKD-dependent phosphorylation of MARCKS, which releases MARCKS from the plasma membrane into the cytoplasm, where it is in turn dephosphorylated by a protein phosphatase 2A (PP2A) that is activated by PKG. This dephosphorylation may allow MARCKS to regain its membrane-binding capability, enabling its attachment to membranes of cytoplasmic mucin granules, In addition, MARCKS interacts with actin and myosin in the cytoplasm and thus may be able to tether the granules to the cellular contractile apparatus, thus, mediating subsequent granule movement and exocytosis. Interestingly, secretion of the inflammatory mediatory MPO from neutrophils may also be maximized by activation of both PKC and PKG (as illustrated in FIGs. 11-15). And it is believed that MARCKS serves as the point of convergence for coordinating actions of these two protein kinases that control secretion from membrane-bound compartments in inflammatory cells (i.e. secretion of MPO from neutrophils),

Transformed cell lines of airway epithelium tend to contain altered signaling pathways, and cell lines or nondifferentiated cells may not respond to exogenous stimuli in a manner similar to differentiated cells *in vivo.* The NHBE cells utilized in the present study were cultured at the air/liquid interface, resulting in fully differentiated primary cell cultures that maintained a well documented structure and function similar to *in vivo* studies. *See,* Krunkosky et al. *supra;* Adler et al., Am. Respir, Cell Mol. Bid 2, 145-154 (1990); Kaartinen et al., In Vitro Cell, Dev. Biol. Anim. 29A, 481-492 (1993); Gray et al., Am. J. Respir. Cell Mol. Biol. 14, 104-112 (1996). This air/liquid methodology to culture airway epithelial cells was developed several years ago to provide an *in vitro* model system to study mechanisms involved in various cellular processes in airway epithelium. The cell cultures contain secretory cells as well as ciliated and basal cells. Results obtained from this culture system are relevant to the response of cells *in vivo* as the heterogeneous cell-cell contacts and polarized epithelial structure are maintained, which likely influence cell behavior *in situ.* Although MARCKS is likely present in non-secretory cells also, the clear and rapid causal associations between modifications of MARCKS and secretory outcomes suggest that mucin secretion is the direct effect of the MARCKS-related molecular events occurring within the secretory cells.

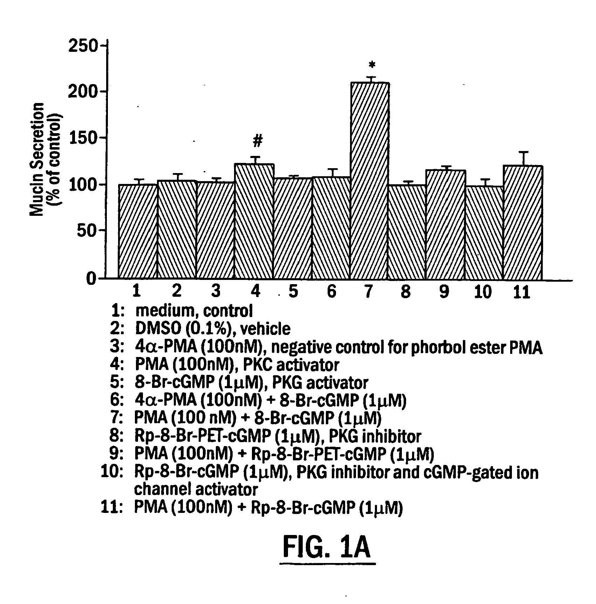

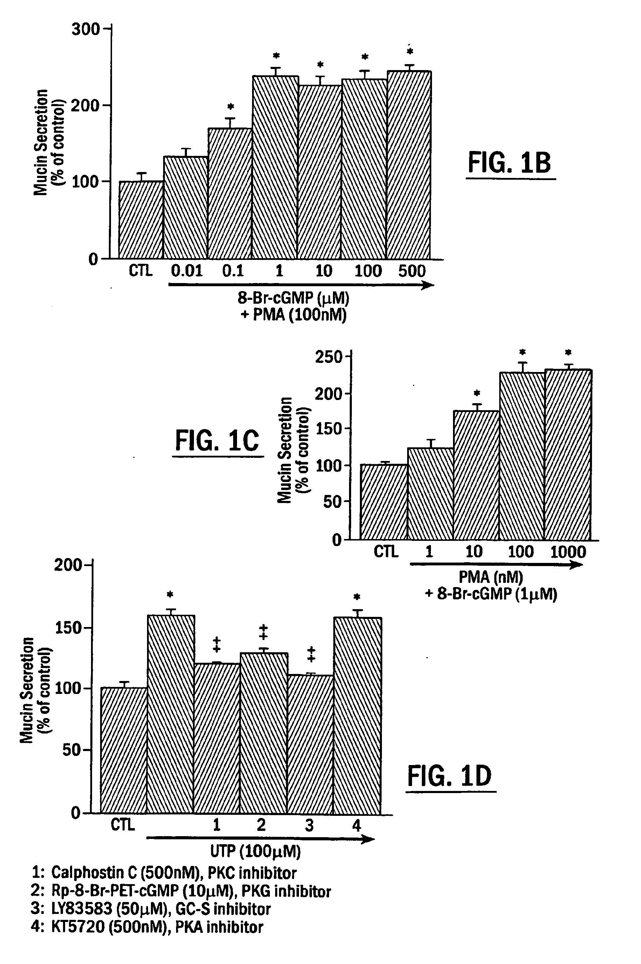

The inventors have demonstrated concurrent activation of both PKC and PKG was able to enhance mucin secretion from differentiated NHBE cells, and that activation of either kinase alone may not be sufficient to elicit a robus secretory response. Similarly, secretion of the inflammatory mediator MPO from canine or human neutrophils was enhanced by concurrent activation of both PKC and PKG, while activation of either kinase alone was insufficient to induce a maximal secretory response. An enhanced secretory response to PMA alone was documented in NHBE cells (FIG. 1, *column 4)* and in neutrophils (FIG. 11), although the magnitude of the response was much less than that observed by others in a rat goblet-like cell line. *See*, Abdullah et al, *supra.* In addition, although it was reported previously that a cGMP analogue could induce significant mucin secretion from cultured guinea pig tracheal epithelial cells (Fischer et al., *supra),* it should be noted that this response did not reach significant levels until 8 h of exposure, A secretory response with such a long lag period is unlikely to be a direct effect and probably involves *de novo* protein synthesis as opposed to release of preformed and stored cytoplasmic granules. Nevertheless, the apparent synergistic effect involving cooperative activation of both PKC and PKG may suggest a complex and stringent signaling mechanism mediating mucin secretion and/or inflammatory mediators. Applicants note that the pathway disclosed below was used to study inflammatory mediator release from neutrophils and is likely the same pathway as that used to study goblet cell secretions.

As stated above, the present invention may be used in a pharmaceutical formulation. In certain embodiments, the drug product is present in a solid pharmaceutical composition that may be suitable for oral administration. A solid composition of matter according to the present invention may be formed and may be mixed with and/or diluted by an excipient. The solid composition of matter also may be enclosed within a carrier, which may be, for example, in the form of a capsule, sachet, tablet, paper, or other container. When the excipient serves as a diluent, it may be a solid, semi-solid, or liquid material that acts as a vehicle, carrier, or medium for the composition of matter.

Various suitable excipients will be understood by those skilled in the art and may be found in the National Formulary, 19: 2404-2406 (2000), the disclosure of pages 2404 to 2406 being incorporated herein in their entirety. Examples of suitable excipients include, but are not limited to, starches, gum arabic, calcium silicate, microcrystalline cellulose, methacrylates, shellac, polyvinylpyrrolidone, cellulose, water, syrup, and methylcellulose. The drug product formulations additionally can include lubricating agents such as, for example, talc, magnesium stearate and mineral oil; wetting agents; emulsifying and suspending agents; preserving agents such as methyl- and propyl hydroxybenzonates; sweetening agents' or flavouring agents. Polyols, buffers, and inert fillers also may be used. Examples of polyols include, but are not limited to, mannitol, sorbitol, xylitol, sucrose, maltose, glucose, lactose, dextrose, and the like. Suitable buffers include, but are not limited to, phosphate, citrate, tartarate, succinate, and the like. Other inert fillers that may be used include those that are known in the art and are useful in the manufacture of various dosage forms. If desired, the solid formulations may include other components such as bulking agents and/or granulating agents, and the like. The drug products of the invention may be formulated so as to provide quick, sustained, or delayed release of the active ingredient after administration to the patient by employing procedures well known in the art.

To form tablets for oral administration, the composition of matter of the present invention may be made by a direct compression process. In this process, the active drug ingredients may be mixed with a solid, pulverant carrier such as, for example, lactose, saccharose, sorbitol, mannitol, starch, amylopectin, cellulose derivatives or gelatin, and mixtures thereof, as well as with an antifriction agent such as, for example, magnesium stearate, calcium stearate, and polyethylene glycol waxes. The mixture may then be pressed into tablets using a machine with the appropriate punches and dies to obtain the desired tablet size. The operating parameters of the machine may be selected by the skilled artisan. Alternatively, tablets for oral administration may be formed by a wet granulation process. Active drug ingredients may be mixed with excipients and/or diluents. The solid substances may be ground or sieved to a desired particle size. A binding agent may be added to the drug. The binding agent may be suspended and homogenized in a suitable solvent. The active ingredient and auxiliary agents also may be mixed with the binding agent solution. The resulting dry mixture is moistened with the solution uniformly. The moistening typically causes the particles to aggregate slightly, and the resulting mass is pressed through a stainless steel sieve having a desired size. The mixture is then dried in controlled drying units for the determined length of time necessary to achieve a desired particle size and consistency. The granules of the dried mixture are sieved to remove any powder. To this mixture, disintegrating, antifriction, and/or anti-adhesive agents may be added. Finally, the mixture is pressed into tablets using a machine with the appropriate punches and dies to obtain the desired tablet size. The operating parameters of the machine may be selected by the skilled artisan.

If coated tables are desired, the above prepared core may be coated with a concentrated solution of sugar or cellulosic polymers, which may contain gum arabic, gelatin, talc, titanium dioxide, or with a lacquer dissolved in a volatile organic solvent or a mixture of solvents. To this coating various dyes may be added in order to distinguish among tablets with different active compounds or with different amounts of the active compound present. In a particular embodiment, the active ingredient may be present in a core surrounded by one or more layers including enteric coating layers.

Soft gelatin capsules may be prepared in which capsules contain a mixture of the active ingredient and vegetable oil. Hard gelatin capsules may contain granules of the active ingredient in combination with a solid, pulverulent carrier, such as, for example, lactose, saccharose, sorbitol, mannitol, potato starch, corn starch, amylopectin, cellulose derivatives, and/or gelatin.

Liquid preparations for oral administration may be prepared in the form of syrups or suspensions, e.g., solutions containing an active ingredient, sugar, and a mixture of ethanol, water, glycerol, and propylene glycol. If desired, such liquid preparations may comprise one or more of following: coloring agents, flavoring agents, and saccharin. Thickening agents such as carboxymethylcellulose also may be used.

In the event that the above pharmaceuticals are to be used for parenteral administration, such a formulation may comprise sterile aqueous injection solutions, non-aqueous injection solutions, or both, comprising the composition of matter of the present invention. When aqueous injection solutions are prepared, the composition of matter may be present as a water soluble pharmaceutically acceptable salt. Parenteral preparations may contain anti-oxidants, buffers, bacteriostats, and solutes which render the formulation isotonic with the blood of the intended recipient. Aqueous and non-aqueous sterile suspensions may comprise suspending agents and thickening agents. The formulations may be presented in unit-dose or multi-dose containers, for example sealed ampules and vials. Extemporaneous injection solutions and suspensions may be prepared from sterile powders, granules and tablets of the kind previously described.

The composition of matter also may be formulated such that it may be suitable for topical administration (*e.g*., skin cream). These formulations may contain various excipients known to those skilled in the art. Suitable excipients may include, but are not limited to, cetyl esters wax, cetyl alcohol, white wax, glyceryl monostearate, propylene glycol, monostearate, methyl stearate, benzyl alcohol, sodium lauryl sulfate, glycerin, mineral oil, water, carbomer, ethyl alcohol, acrylate adhesives, polyisobutylene adhesives, and silicone adhesives.

Having now described the invention, the same will be illustrated with reference to certain examples, which are included herein for illustration purposes only, and which are not intended to be limiting of the invention.

### EXAMPLES

### Mucin Hypersecretion from NHBE Cells Involves Activation of Both PKC and PKG

To determine the potential role of PKC and/or PKG in the mucin secretory process, NHBE cells were exposed to the following two specific protein kinase activators: the phorbol ester, phorbol 12-myristate 13-acetate (PMA), for activation of PKC, and the nonhydrolyzable cGMP analogue, 8-Br-cGMP, for activation of PKG. Preliminary studies examining mucin secretion in response to PMA stimulation at various concentrations for different times (up to 1 µM for 2 h) indicated that activation of PKC alone did not induce significant mucin secretion from NHBE cells, although a moderate secretory response was repeatedly observed at PMA concentrations higher than 100 nM (0.05 <*p* < 0.1). Also, the cells did not respond to the cGMP analogues at concentrations as high as 500 µM for up to 2 h of exposure. However, a combination of PMA + 8-Br-cGMP, affecting dual activation of PKC and PKG, provoked a rapid increase in secretion, approximately doubling it within 15 min of exposure (FIG. 1A). This secretory response induced by PMA + 8-Br-cGMP was concentration-dependent, with maximal stimulation at 100 nM PMA + 1 µM 8-BrcGMP (FIGS. 1B and 1C). In FIGS. 1A, 1B and 1C, NHBE cells were exposed to indicated reagent(s) or medium alone *(CTL)* for 15 min. In FIG, 1D, NHBE cells were preincubated with the indicated inhibitor for 15 min and then stimulated with 100 µM UTP for 2 h. Secreted mucin in response to the treatment was collected and assayed by ELISA. Data are presented as mean ± S.E. *(n* = 6 at each point). The * stands for significantly different from medium control *(p <0.05);* # stands for different from medium control (0.05 *<p* < 0.1); and t stands for significantly different from UTP stimulation (*p* < *0.05*)

UTP is a well defined pathophysiologically relevant mucin secretagogue. Lethem et al., Am. J. Respir. Cell Mot Biol. 9, 315-322 (1993). The present invention further demonstrates that UTP, at various concentrations, preferably 40 to 140 µM, may induce a significant increase in mucin secretion from NHBE cells after a 2-h exposure. To determine whether PKC and PKG were involved in regulation of mucin secretion in response to a pathophysiological stimulus, effects of PKC/PKG inhibitors on UTP-induced mucin secretion were investigated. NHBE cells were preincubated with various inhibitors for 15 min and then exposed to UTP (100 µM) plus the inhibitor for 2 h. The secreted mucin was measured by ELISA. The results indicated that mucin secretion provoked by UTP may require both PKC and PKG activities, as the secretory response was attenuated independently by the PKC inhibitor calphostin C (500 nM), the PKG inhibitor Rₚ-8-Br-PET-cGMP (10 µM), or the soluble guanylyl cyclase (GC-S) inhibitor LY83583 (50 µM) but likely not by the protein kinase A (PKA) inhibitor KT5720 (500 nM) (FIG. ID). Apparently, mucin secretion in NHBE cells may be regulated by a signaling mechanism involving both PKC and PKG.

To address involvement of PKG in the secretory process, S-Br-cGMP was utilized in these studies. Although the primary physiological effect of 8-Br-cGMP is to activate PKG, it also has been reported to act as an agonist for cGMP-gated ion channels in some cells and, at high concentrations, to cross-activate PKA. To preclude the possibility that cGMP-gated ion channels and/or PKA may play a role in mucin secretion by NI-IBE cells, Rₚ-8-Br-cGMP, a unique cGMP analogue that can activate cGMP-gated ion channels similar to 8-Br-cOMP but inhibit PKG activity, was used as an agonist to distinguish the effects of PKG and cGMP-gated ion channels on mucin release. As illustrated in the figures, particularly, FIG. 1A *(column 11*), Rₚ-8-BrcGMP did not enhance mucin secretion when added to the cells with PMA. Likewise, the specific PKA inhibitor, KT5720 (500 nM), did not affect mucin secretion induced by either PMA + 8-Br-cGMP or UTP (FIG. 1D, *column 4).* These studies may negate the possibility that cGMP-gated ion channels or PKA are associated with mucin secretion, indicating that activation of PKG in NHBE cells is the mechanism whereby 8-Br-cGMP contributes to enhanced secretion. Furthermore, because UTP-induced mucin hypersecretion can be attenuated by the soluble guanylyl cyclase (GC-S) inhibitor LY83583, it is likely that activation of PKG occurs via the signaling pathway of nitric oxide (NO) → GC-S→ cGMP→ PKG, as illustrated previously in differentiated guinea pig tracheal epithelial cells *in vitro.*

Given the participation of both PKC and PKG in the mucin secretory process, the present invention examines potential intracellular substrates of these enzymes that could play a role in signaling events downstream of the kinase activation. Numerous intracellular substrates can be phosphorylated by PKC or PKG, and phosphorylation by PKC of one such substrate, MARCKS protein, seemed to be of particular interest. MARCKS phosphorylation has been observed to correlate with a number of cellular processes involving PKC signaling and cytoskeletal contraction, such as cell movement, mitogenesis, and neural transmitter release. Because the dynamic process of secretion requires both kinase activation and translocation of intracellular granules to the cell periphery, MARCKS appeared to be a candidate for a mediator molecule connecting PKC/PKG activation and mucin granule exocytosis.

### MARCKS Is a Key Molecule Linking PKC/PKG Activation to Mucin Secretion in NHBE Cells

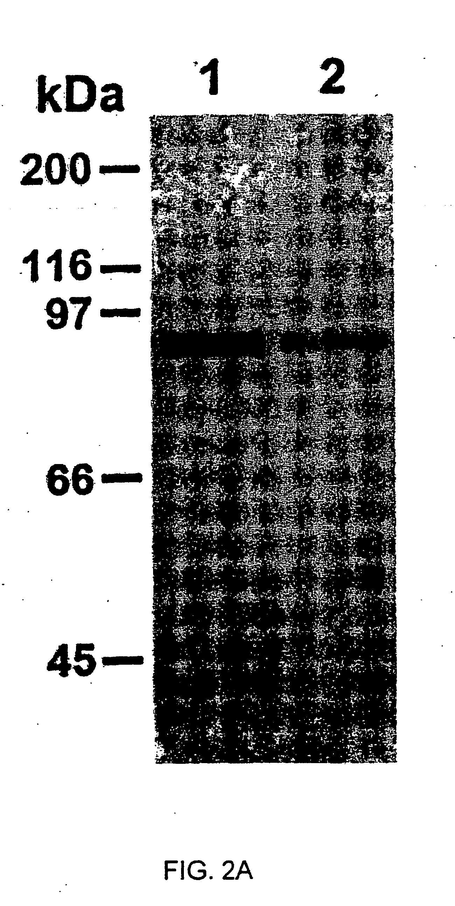

To address the signaling mechanism downstream of protein kinase activation, MARCKS protein, a specific cellular substrate of PKC that might play a role in linking kinase activation to granule release was studied. First, the presence of MARCKS in NHBE cells by [³H]myristic acid-labeled immunoprecipitation assay was confirmed. As illustrated in FIG. 2A, MARCKS was expressed in NHBE cells, and the majority of this protein was membrane-associated under unstimulated conditions. In FIG. 2A, cells were labeled with [³H]myristie acid overnight and the membrane (lane 1) and the cytosol (lane 2) fractions were then isolated by differential centrifugation. A role for MARCKS as a key regulatory component of the mucin secretory pathway may be demonstrated in three different ways.

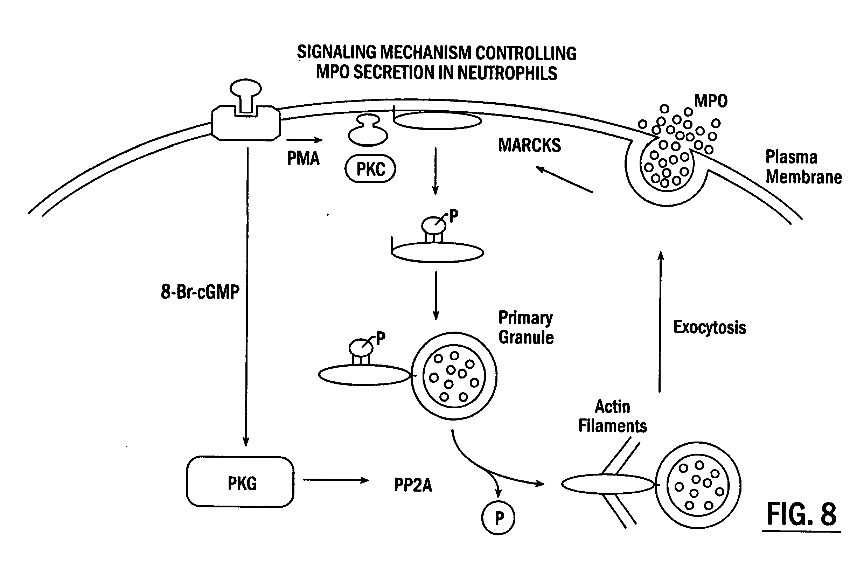

As stated above, direct involvement of MARCKS in mucin secretion by NHBE cells may be demonstrated by three separate lines of evidence. First, mucin secretion in response to stimulation by PMA 8-Br-cGMP or UTP was inhibited in a concentration-dependent manner by the MANS peptide, which had the amino acid sequence identical to the N-terminal region of MARCKS, whereas the corresponding control peptide (RNS), containing the same amino acid composition but arranged in random order, did not affect secretion. The N-terminal myristoylated domain of MARCKS is known to mediate the MARCKS -membrane association. As indicated in FIG. 8, MARCKS may function as a molecular linker by interacting with granule membranes at its N-terminal domain and binding to actin filaments at its PSD site, thereby tethering granules to the contractile cytoskeleton for movement and exocytosis. FIG. 8 shows a possible mechanism depicting that mucin secretagogue interacts with airway epithelial (goblet) cells and activates two separate protein kinases, PKC and PKG. Activated PKC phosphorylates MARCKS, causing MARCKS translocation from the plasma membrane to the cytoplasm, whereas PKG, activated via the nitric oxide (NO) GC-S cGMP PKG pathway, in turn activates a cytoplasmic PP2A, which dephosphorylates MARCKS. This dephosphorylation stabilizes MARCKS attachment to the granule membranes. In addition, MARCKS also interacts with actin and myosin, thereby linking granules to the cellular contractile machinery for subsequent movement and exocytotic release. The attachment of MARCKS to the granules after it is released into the cytoplasm may also be guided by specific targeting proteins or some other forms of protein-protein interactions in which the N-terminal domain of MARCKS is involved. In either case, the MANS peptide, or an active fragement thereof, comprising at least 6 amino acids, would act to inhibit competitively targeting of MARCKS to the membranes of mucin granules, thereby blocking secretion.

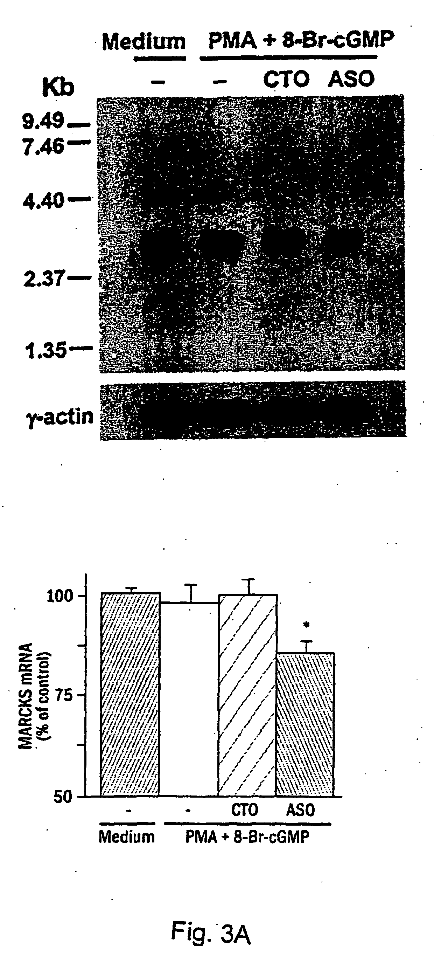

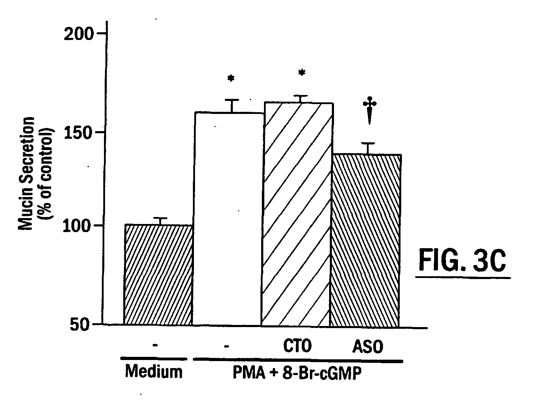

A second test demonstrated the inhibitory effect of a MARCKS-specific antisense oligonucleotide on mucin secretion. As shown in FIGs. 3A-3C, the antisense oligonucleotide down-regulated MARCKS mRNA and protein levels in NHBE cells and substantially attenuated mucin secretion induced by PKC/PKG activation. The inhibition was not as dramatic as that seen with the MANS peptide, which might be due to the high levels of endogenous MARCKS protein in NHBE cells and the relatively long half-life of MARCKS mRNA (tie = 4-6 h). In FIGs. 3A-3C, NHBE cells were treated with the antisense or the control oligonucleotide for 3 days and then stimulated with PMA (100 nM) + 8-Br-cGMP (1 uM) for 15 min. Mucin secretion was analyzed by ELISA. Total RNA and protein were isolated from treated cells. MARCKS mRNA was assessed by Northern hybridization, and protein was assessed by Western blot. In the PMA (100 nM) 8-Br-cGMP (1 um) FIG. 3A is a Northern blot that showed a decrease of-15% in MARCKS inRNA compared with controls in the attached chart; FIG, 3B is Western blot that showed a decrease of -30% in MARCKS protein in the attached graph; and FIG. 3C shows mucin hypersecretion was attenuated significantly by the antisense oligonucleotide, whereas the control oligonucleotide had no effect. Data are presented as mean ± S.E. (*n* = 6 at each point) wherein the * is significantly different from medium control *(p* < 0.05); and the t is significantly different from PMA + 8- Br-cGMP stimulation *(p* < 0.05). Additionally, it is noted that the term CTO is the control oligonucleotide, while the term ASO is an antisense oligonucleotide.

It has been demonstrated that antisense oligonucleotides that are complementary to specific RNAs can inhibit the expression of cellular genes as proteins. See Erickson and Izant, Gene Regulation: Biology Of Antisense RNA And DNA, Vol. 1, Raven Press, New York, 1992. For example, selective inhibition of a p21 gene that differed from a normal gene by a single nucleotide has been reported. Chang et al., Biochemistry 1991,30:8283-8286. Many hypotheses have been proposed to explain the mechanisms by which antisense oligonucleotides inhibit gene expression, however, the specific mechanism involved may depend on the cell type studied, the RNA targeted, the specific site on the RNA targeted, and the chemical nature of the oligonucleotide. Chiang et al., J Biol. Chem. 1991, 266:18162-18171; Stein and Cohen, Cancer Res. 1988, 48:2659-2668.

A third experiment indicated that transfection of HBE1 cells with a PSD-deleted mutant MARCKS resulted in significant repression of mucin secretion induced by PKC/PKG activation. Deletion of the PSD would abolish the ability of MARCKS to bind to actin. As indicated in FIG. 8, by competing with native MARCKS for binding to granule membrane, the PSD-truncated MARCKS could thereby inhibit granule release as it is unable to interact with the actin filaments. Transfection of these cells with the wild-type MARCKS cDNA did not further enhance mucin secretion. Western blot assay showed that the expression level of endogenous MARCKS in HBE1 cells was quite high, comparable with that in NHBE cells, and transfection of wild-type MARCKS cDNA did not lead to notable increases in overall MARCKS protein level in these cells. This may explain why transfection with wild-type MARCKS did not further augment secretion and also why transfection with the PSD-deleted MARCKS only partially hindered muein secretion.

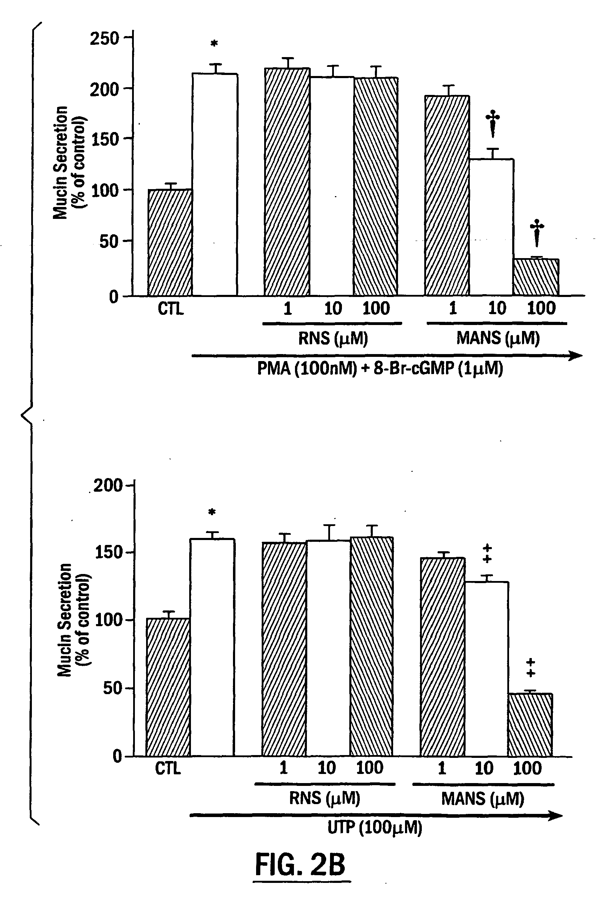

*Peptide Blocking Studies*-- NHBE cells were preincubated with either the MANS or the RNS peptide (1-100 µM) for 15 min, and then PMA (100 nM) + 8- Br-cGMP (1 µM) or UTP (100 µM) was added, and cells were incubated for an additional 15 min or 2 h, respectively. Mucin secretion was measured by ELISA. As shown in FIG. 2B, incubation of NHBE cells with the MANS peptide resulted in a concentration-dependent suppression of mucin secretion in response to PKC/PKG activation or UTP stimulation, whereas the control peptide (RNS) may not have affected secretion at these same concentrations. In FIG. 2B, the MANS peptide blocks mucin hypersecretion induced by PMA + 8-Br-cGMP or UTP in a concentration-dependent manner. NHBE cells were preincubated with the indicated peptide for 15 min and then exposed to PMA (100 µm) + 8-Br-cGMP (1 µm) for 15 min or UTP (100 µm) for 2 h. Mucin secretion was measured by ELISA. Data are presented as mean ± S.E. *(n* = 6 at each point), wherein * is significantly different from medium control *(p* < 0.05); † is significantly different from PMA + 8-Br-cGMP stimulation *(p < 0.05);* and ‡ is significantly different from UTP stimulation (*p* < 0.05). Effects of the MANS peptide were likely not related to cytotoxicity or general repression of cellular metabolic activity, as neither the MANS nor the RNS peptide affected lactate dehydrogenase release or [³H]deoxyglucose uptake by the cells.

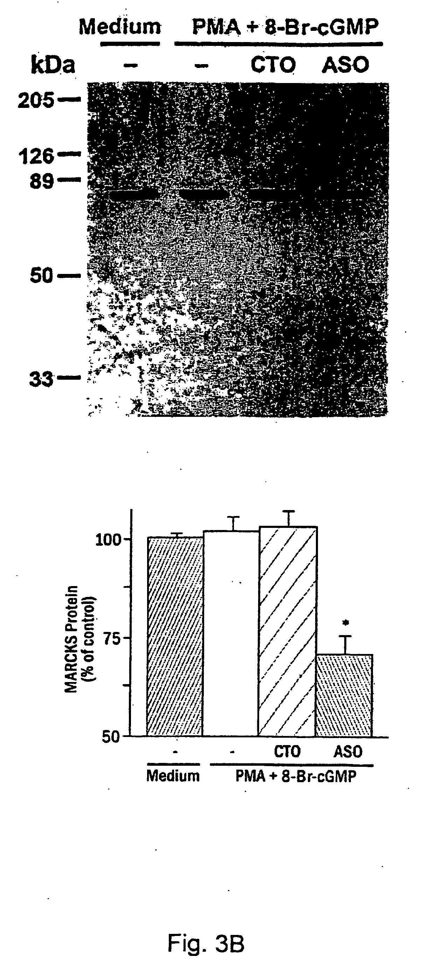

*Antisense Oligonucleotide Studies--* To demonstrate further MARCKS as a key signaling component of the mucin secretory pathway, the effect of an antisense oligonucleotide directed against MARCKS on mucin secretion was examined. As illustrated in FIG. 3, this antisense oligonucleotide down-regulated both mRNA and protein levels of MARCKS in NHBE cells and significantly attenuated mucin secretion induced by PMA + 8-Br-cGMP, whereas a control oligonucleotide had no effect.

### MARCKS Serves as a Convergent Signaling Molecule Mediating Cross-talk of PKC and PKG Pathways

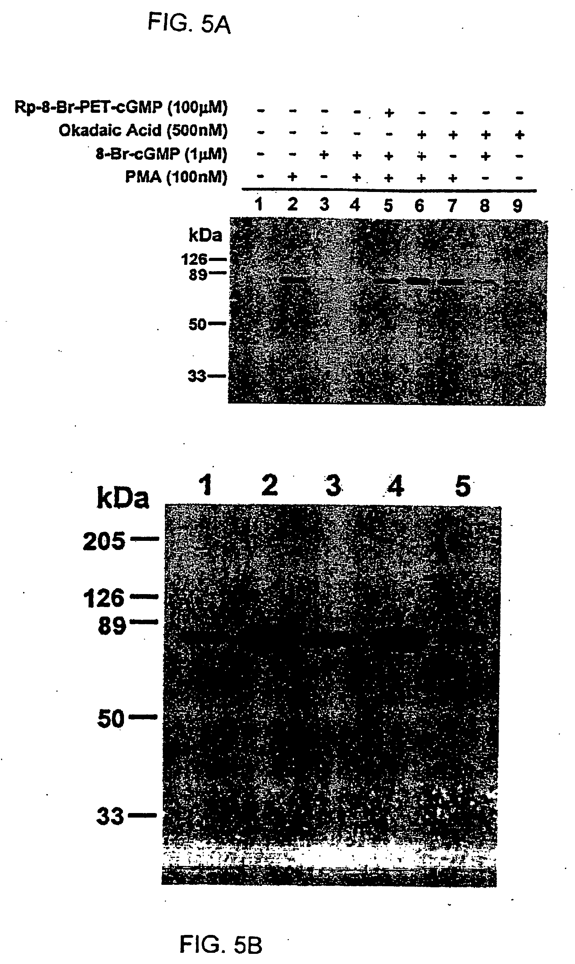

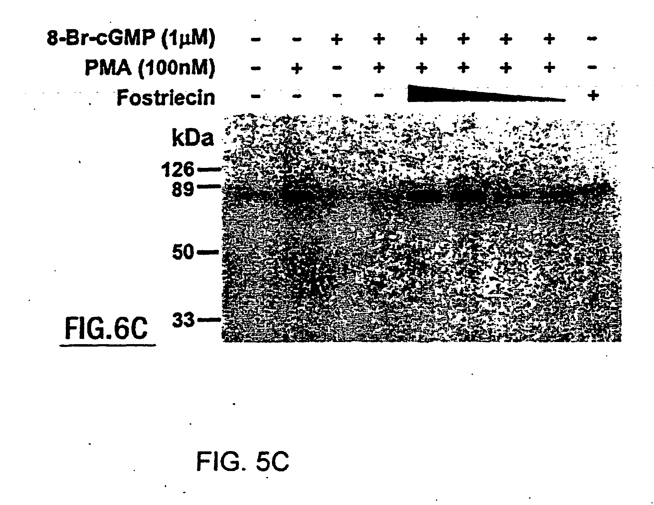

Collectively, the above results demonstrated that MARCKS was involved integrally in the mucin secretory process. Next the present inventors addressed how MARCKS acts as a key regulatory molecule upon which PKC and PKG converge to regulate mucin secretion. As illustrated in FIG. 5, MARCKS was phosphorylated by PKC and consequently translocated from the membrane to the cytoplasm. Here, PKG appeared to induce dephosphorylation of MARCKS (FIG. 5A, lane 4, and FIG. 5B), This dephosphorylation was reversed by the PKG inhibitor Rp -8-Br-PET-cGMP (FIG. 5A, lane 5), indicating the dephosphorylation was specifically PKG-dependent. In FIG. 5, the NHBE cells were labeled with [³²P]orthophosphate a and then exposed to the indicated reagents. MARCKS phosphorylation in response to the treatments was evaluated by immunoprecipitation assay. In FIG. 5A, 8-Br-cGMP reversed MARCKS phosphorylation induced by PMA, and this effect of 8-Br-cGMP could be blocked by Rₚ-8-Br-PET-cGMP (PKG inhibitor) or okadaic acid (PP1/2A inhibitor). For FIG. 5B, PMA-induced phosphorylation of MARCKS was reversed by subsequent exposure of cells to 8-Br-cGMP. *Lane 1*, medium alone for 8 min; *lane 2*, 100 nM PMA for 3 min; *lane 3*, 100 nM PMA for 3 min and then with 1 µM 8-BrcOMP for 5 min; *lane 4,* 100 nM PMA for 8 min; *lane 5*, medium alone for 3 min and then 100 nM PMA + 1 µM 8-Br-cGMP for 5 min. In FIG 5C, 8-Br-cGMP-induced MARCKS dephosphorylation was attenuated by fostriecin in a concentration- dependent manner.

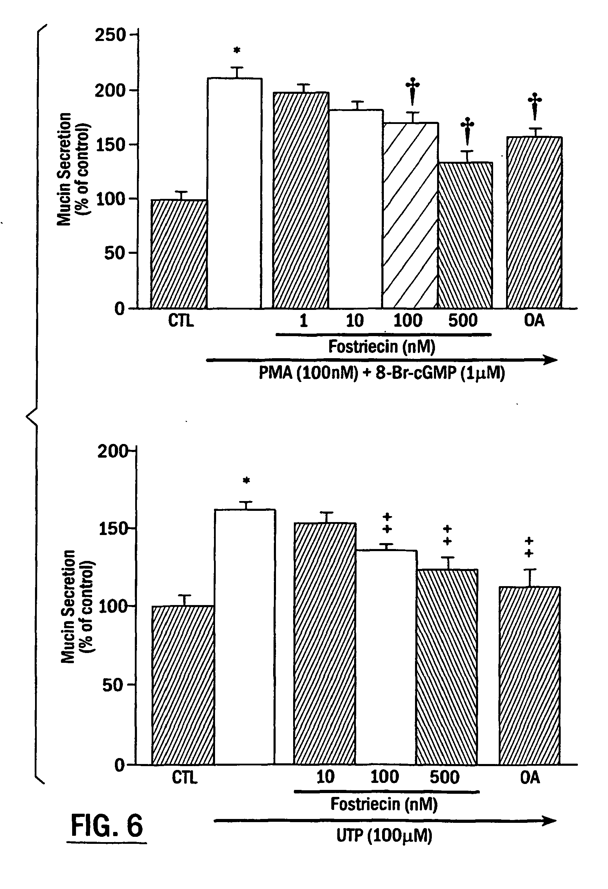

It is believed that PKG acts to dephosphorylate MARCKS via activation of a protein phosphatase. As illustrated in FIG. 5A (lane 6), okadaic acid at 500 nM, a concentration that could inhibit both PP1 and PP2A, blocked PKG-induced dephosphorylation of MARCKS, suggesting that PKG caused dephosphorylation by activating PP1 and/or PP2A. Further studies with fostriecin and direct assay of phosphatase activities indicated that only PP2A was activated by PKG and was responsible for removal of the phosphate groups from MARCKS (FIG. *5C**).* It is likely that either okadaic acid or fostriecin, at concentrations that inhibited PKGinduced dephosphorylation of MARCKS, attenuated mucin secretion induced by PMA + 8-Br-cGMP or UTP as exhibited in FIG. 6. FIG. 6 helps to demonstrate that PP2A is an essential component of the mucin secretory pathway. NHBE cells were preincubated with the indicated concentration of fostriecin, okadaic acid (500 nM), or medium alone for 15 min and then stimulated with PMA (100 nM) + 8-Br-cGMP (1µM) for 15 min or with UTP (100µM) for 2 h. Secreted mucin was measured by ELISA. Data are presented as mean ± S.E. *(n*= 6 at each point) wherein * stands for significantly different from medium control *(p* < 0.05); † stands for significantly different from PMA + 8-Br-cGMP stimulation *(p* < 0.05); and ‡ stands for significantly different from UTP stimulation *(p* < 0,05), Thus, dephosphorylation of MARCKS by a PKG-activated PP2A appears to be an essential component of the signaling pathway leading to mucin granule exocytosis.

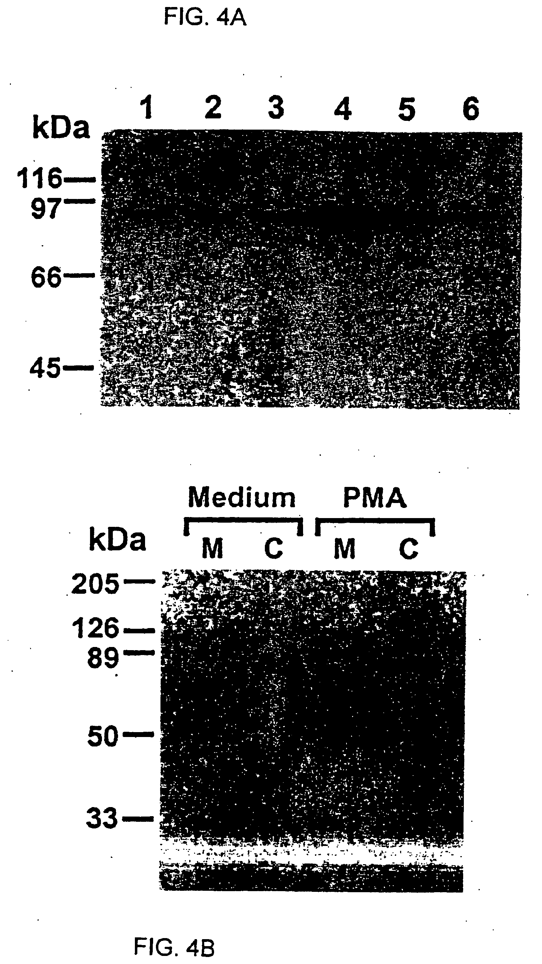

To reveal molecular events by which MARCKS links kinase activationto mucin secretion, phosphorylation of MARCKS in response to PKC/PKG activation was investigated in depth. As illustrated in FIG. 4A, PMA (100 nM) likely induced a significiant ncrease (3 - 4 fold) in MARCKS phosphorylation in NHBE cells, and this phosphorylation was attenuated by the PKC inhibitor calphostin C (500 nm). Once phosphorylated, MARCKS was translocated from the plasma membrane to the cytoplasm (FIG. 4B). More specifically, FIG. 4A shows the activation of PKC results in MARCKS phosphorylation in NI-IBE cells, Cells were labeled with [³²P]orthophosphate for 2 h and then exposed to the stimulatory and/or inhibitory reagents. MARCKS phosphorylation in response to the treatments was evaluated by immunoprecipitation as described. *Lane 1*, medium control; *lane 2,* the vehicle, 0.1% Me₂SO; *lane 3,* 100 nM 4α-PMA; *lane 4,* 100 nM PMA; *lane 5,* 100 nM PMA + 500 nM calphostin C; *lane 6,* 500 nM calphostin C. FIG 4B demonstrates phosphorylated MARCKS is translocated from the plasma membrane to the cytoplasm. ³²P-Labeled cells were exposed to PMA (100 nM) or medium alone for 5 min, and then the membrane and the cytosol fractions were isolated. Activation of PKG by 8-Br-cGMP (1 µM), another kinase activation event necessary for provoking mucin secretion, did not lead to MARCKS phosphorylation, but, in fact, the opposite effect was observed: MARCKS phosphorylation induced by PMA was reversed by 8- Br-cGMP (FIG. 5A). This effect of 8-Br-cGMP was not due to suppression of PKC activity, as the PMA-induced phosphorylation could be reversed by subsequent addition of 8-Br-cGMP to the cells (FIG. 5B), Therefore, PKG activation likely results in dephosphorylation of MARCKS.

Further investigation demonstrated that PKG-induced MARCKS dephosphorylation was blocked by 500 nM okadaic acid, a protein phosphatase (type 1 and/or 2A (PP1/2A)) inhibitor (FIG, 5A, lane 6). Thus, it appeared that the dephosphorylation was mediated by PP1 and/or PP2A. To define the subtype of protein phosphatase involved, a novel and more specific inhibitor of PP2A, fostriecin (IC₅₀ = 3.2 nM), was utilized in additional phosphorylation studies. As illustrated in FIG. 5C, fostriecin inhibited PKG-induced MARCKS dephosphorylation in a concentration-dependent manner (1-500 nM), suggesting that PKG induced the dephosphorylation via activation of PP2A. To confirm further activation of PP2A by PKG in NHBE cells, cytosolic PP1 and PP2A activities were determined after exposure of the cells to 8-Br-cGMP. PP2A activity was increased approximately 3- fold (from 0.1 to 0.3 nmol/min/mg proteins, *p* <*0.01*) at concentrations of 8-BrcGMP as low as 0.1 µM, whereas PP1 activity remained unchanged. This data indicates that PP2A may be activated by PKG and is responsible for the dephosphorylation of MARCKS. Accordingly, this PP2A activity appeared critical for mucin secretion to occur; when PKG-induced MARCKS dephosphorylation was blocked by okadaic acid or fostriecin, the secretory response to PKC/PKG activation or UTP stimulation was ameliorated (FIG. 6).

### MARCKS Associates with Actin and Myosin in the Cytoplasm

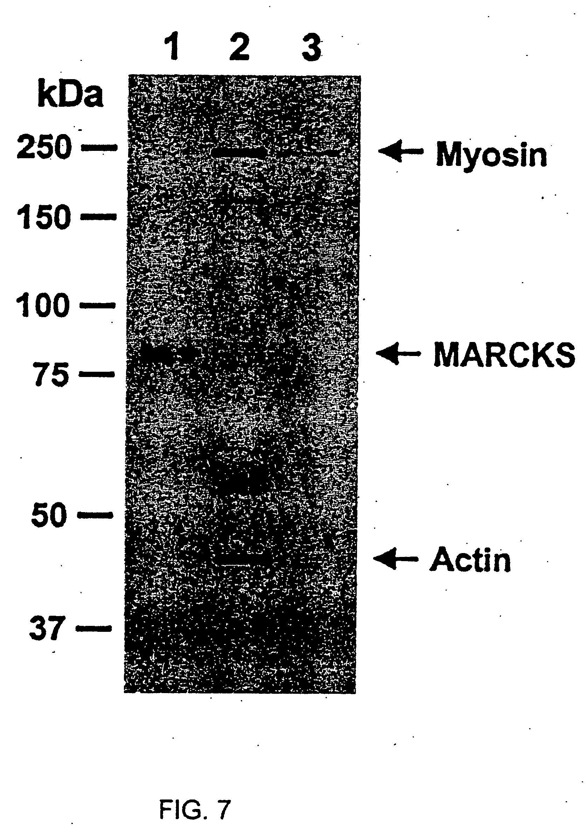

FIG. 7 depicts a radio labeled immunoprecipitation assay which reveals that MARCKS may associate with two other proteins (~200 and ~40 kDa) in the cytoplasm. In FIG. 7 NHBE cells were labeled with [³H] leucine and [³H] proline overnight, and the membrane and the cytosol fractions were prepared as described under "Experimental Procedures." Isolated fractions were precleared with the nonimmune control antibody (6F6). The cytosol was then divided equally into two fractions and used for immunoprecipitation carried out in the presence of 10 gm cytochalasin D (Biomol, Plymouth Meeting, PA) with the anti-MARCKS antibody 2F12 *(lane 2)* and the nonimmune control antibody 6F6 *(lane 3)*, respectively. MARCKS protein in the membrane fraction was also assessed by immunoprecipitation using the antibody 2F12 *(lane 1).* The precipitated protein complex was resolved by 8% SDS-polyacrylamide gel electrophoresis and visualized by enhanced autoradiography. MARCKS appeared to associate with two cytoplasmic proteins with molecular masses of ~200 and ~40 kDa, respectively. These two MARCKS-associated proteins were excised from the gel and analyzed by matrix- assisted laser desorption ionization/time of flight mass spectrometry/internal sequencing (the Protein/DNA Technology Center of Rockefeller University, New York). The obtained peptide mass and sequence data were used to search protein databases via Internet programs ProFound and MS-Fit. Results indicate that they are myosin (heavy chain, non-muscle type A) and actin, respectively. Matrix-assisted laser desorption ionization/time of flight mass spectrometry/internal sequence analysis indicates that these two MARCKS-associated proteins were myosin (heavy chain, non- muscle type A) and actin, respectively.

These studies suggest a new paradigm for the signaling mechanism controlling exocytotic secretion of airway mucin granules as well as providing what is believed to be the first direct evidence demonstrating a specific biological function of MARCKS in a physiological process. MARCKS serves as a key mediator molecule regulating mucin granule release in human airway epithelial cells. It is believed that elicitation of airway mucin secretion requires dual activation and synergistic action of PKC nad PKG. Activated PKC phosphorylates MARCKS, resulting in translocation of MARCKS from the inner face of the plasma membrane into the cytoplasm, Activation of PKG in turn activates PP2A, which dephosphorylates MARCKS in the cytoplasm. Because the membrane association ability of MARCKS is dependent on its phosphorylation state this dephosphorylation may allow MARCKS to regain its membrane-binding capability and may enable MARCKS to attach to membranes of cytoplasmic mucin granules. By also interacting with actin and myosin in the cytoplasm (FIG. 7), MARCKS may then be able to tether granules to the cellular contractile apparatus, mediating granule movement to the cell periphery and subsequent exocytotic release. The wide distribution of MARCKS suggests the possibility that this or a similar mechanism may regulate secretion of membrane-bound granules in various cell types under normal or pathological conditions.

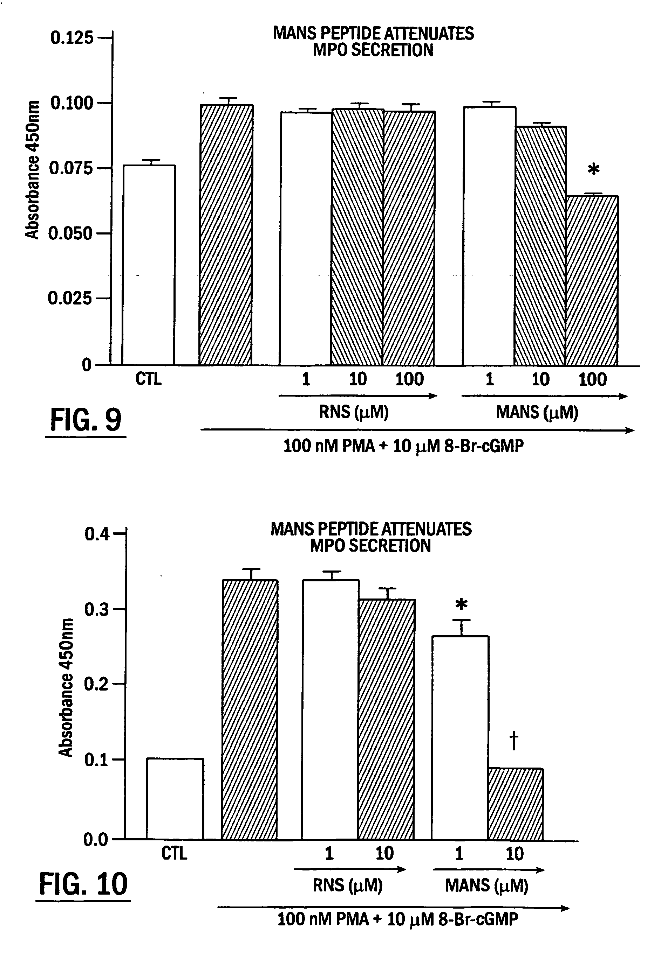

Also envisaged is a new method for blocking any cellular secretory process, especially those releasing inflammatory mediators from inflammatory cells, whose stimulatory pathways involve the protein kinase C (PKC) substrate MARCKS protein and release of contents from membrane-bound vesicles. Specifically, the inventors have shown that stimulated release of the inflammatory mediator myloperoxidase from human (FIG. 9) or canine (FIG, 10) neutrophils can be blocked in a concentration-dependent manner by the MANS peptide. Specifically, FIG. 9 shows isolated neutrophils that were stimulated to secrete myloperoxidase (MPO) with 100nM PMA and 101µM 8-Br-cGMP. 100 µM MANS peptide decreased secretion of MPO to control levels (* = p<0.05). 10 µM MANS causes a slight decrease in MPO secretion. 10 or 100 µM of a control peptide (RNS) has no effect on MPO secretion. In FIG. 10, isolated neutrophils were stimulated to secrete myloperoxidase (MPO) with 100nM PMA and 10 µM 8-Br-cGMP. 100 µM MANS peptide decreased secretion of MPO to control levels (* = p<0.05). 10 µM MANS causes a slight decrease in MPO secretion. 10 or 100 µM of a control peptide (RNS) has no effect on MPO secretion, Thus, the peptide may be used therapeutically to block the release of mediators of inflammation secreted from infiltrating inflammatory cells in any tissues. Many of these released mediators are responsible for the extensive tissue damage observed in a variety of chronic inflammatory diseases (*i.e*., respiratory diseases such as asthma, chronic bronchitis and COPD, inflammatory bowel diseases including ulcerative colitis and Crohn's disease, autoimmune diseases, skin diseases such as rosacea, eczema, and severe acne, arthritic and pain syndromes such as rheumatoid arthritis and fibromyalgia). This invention may be useful for treating diseases such as arthritis, chronic bronchitis, COPD and cystic fibrosis. This invention is accordingly useful for the treatment in both human and animal diseases, especially those affecting equines, canines, felines, and other household pets.

Figures 11-15 show MPO secretion for both humans and canines. In allof these experiments, isolated neutrophils were stimulated with LPS at a concentration of 1x 10⁻⁶M for 10 minutes at 37°C prior to adding the stimuli as indicated in the figures. The LPS primes the cells so they can respond to a secretagogue.

### Methods and Materials

*NHBE Cell Culture--* Expansion, cryopreservation, and culture of NHBE cells in the air/liquid interface were performed as described previously. *See,* Krunkosky *et al.* Briefly, NHBE cells (Clonetics, San Diego, CA) were seeded in vented T75 tissue culture flasks (500 cells/cm²) and cultured until cells reached 75 - 80% confluence, Cells were then dissociated by trypsin/EDTA and frozen as passage- 2. Air/liquid interface culture was initiated by seeding passage-2 cells (2 x 10⁴ cells/cm²) in TRANSWELL® clear culture inserts (Costar, Cambridge, MA) that were thinly coated with rat tail collagen, type 1 (Collaborative Biomedical, Bedford, MA). Cells were cultured submerged in medium in a humidified 95% air, 5% CO2 environment for 5-7 days until nearly confluent. At that time, the air/liquid interface was created by removing the apical medium and feeding cells basalaterally. Medium was renewed daily thereafter. Cells were cultured for an additional 14 days to allow for full differentiation.

*Measurement of Mucin Secretion by ELISA--* Before collection of "base line" and "test" mucin samples, the accumulated mucus at the apical surface of the cells was removed by washing with phosphate-buffered saline, pH 7.2. To collect the base-line secretion, cells were incubated with medium alone, and secreted mucin in the apical medium was collected and reserved. Cells were rested for 24 h and then exposed to medium containing the selected stimulatory and/or inhibitory reagents (or appropriate controls), after which secreted mucin was collected and reserved as the test sample. Incubation times for the base line and the test were the same but varied depending on the test reagent utilized. Both base line and test secretions were analyzed by ELISA using an antibody capture method as known in the art. *See, e.g.,* Harlow et al., Antibodies: A Laboratory Manual, pp. 570-573, Cold Spring Harbor Laboratory, Cold Spring Harbor, NY (1988). The primary antibody for this assay was 17Q2 (Babco, Richmond, CA), a monoclonal antibody that reacts specifically with a carbohydrate epitope on human airway mucins. The ratio of test/base-line mucin, is similar to a "secretory index", was used to quantify mucin secretion, allowing each culture dish to serve as its own control and thus, minimizing deviation caused by variability among culture wells. Wright et al., Am. J. Physiol. 271, L854-L861 (1996). Levels of mucin secretion were reported as percentage of the medium control.

*Radiolabeled Immunoprecipitation Assay--* When labeling with [³²P]phosphate, cells were preincubated for 2 h in phosphate-free Dulbecco's modified Eagle's medium containing 0.2% bovine serum albumin and then labeled with 0.1 mCi/ml [³²P]orthophosphate (9000 Ci/mmol, PerkinElmer Life Sciences) for 2 h. For labeling with [³H]myristic acid or ³H -amino acids, cells were incubated overnight in medium containing 50 µCi/ml [³H]myristic acid (49 Ci/mmol, PerkinElmer Life Sciences) or 0.2 mCi/ml [³H]leucine (159 Ci/mmol, PerkinElmer Life Sciences) plus 0.4 mCi/ml [³H]proline (100 Ci/mmol, PerkinElmer Life Sciences). Following labeling, cells were exposed to stimulatory reagents for 5 min. When an inhibitor was used, cells were preincubated with the inhibitor for 15 min prior to stimulation. At the end of the treatments, cells were lysed in a buffer containing 50 mM Tris-HCl (pH 7.5), 150 mM NaCl, 1 mM EDTA, 10% glycerol, 1% Nonidet P-40, 1 mM phenylmethylsulfonyl fluoride, 1 mM benzamidine, 10 µg/ml pepstatin A, and 101.µg/ml leupeptin. Trichloroacetic acid precipitation and scintillation counting may determine the radiolabeling efficiency in each culture. Immunoprecipitation of MARCKS protein was carried out according to the method of Spizz and Blackshear using cell lysates containing equal counts/min. Spizz et al., J. Biol. Chem. 271, 553562 (1996). Precipitated proteins were resolved by 8% SDS-polyacrylamide gel electrophoresis and visualized by autoradiography. Anti-human MARCKS antibody (2m) and nonimmune control antibody (6F6) were used in this assay.

To assess MARCKS or MARCKS-associated protein complexes in different subcellular fractions, radiolabeled and treated cells were scraped into a homogenization buffer (50 mM Tris-HCl (pH 7.5), 10 mM NaCl, 1 mM EDTA, 1 mM phenylmethylsulfonyl fluoride, 1 mM benzamidine, 10 µg/ml pepstatin A, 10 µg/ml leupeptin) and then disrupted by nitrogen cavitation (800 pounds/square inch for 20 min at 4 °C). Cell lysates were centrifuged at 600 x g for 10 min at 4 °C to remove nuclei and unbroken cells. Post-nuclear supernatants were separated into membrane and cytosol fractions by ultracentrifugation at 400,000 x g for 30 min at 4°C. The membrane pellet was solubilized in the lysis buffer by sonication. Immunoprecipitation was then carried out as described above.

*MARCKS-related Peptides--* Both the myristoylated N-terminal sequence (MANS) and the random N-terminal sequence (RNS) peptides were synthesized at Genemed Synthesis, Inc. (San Francisco, CA), then purified by high pressure liquid chromatography (>95% pure), and confirmed by mass spectroscopy with each showing one single peak with an appropriate molecular mass. The MANS peptide consisted of sequence identical to the first 24 amino acids of MARCKS, *i,e*. the myristoylated N-terminal region that mediates MARCKS insertion into membranes, MA-GAQFSKTAAKGEAAAERPGEAAVA (SEQ ID NO: I (where MA = N-terminal myristate chain). The corresponding control peptide (RNS) contained the same amino acid composition as the MANS but arranged in random order, MA-GTAPAAEGAGAEVKRASAEAKQAF (SEQ ID NO: 2). The presence of the hydrophobic myristate moiety in these synthetic peptides enhances their permeability to the plasma membranes, enabling the peptides to be taken up readily by cells. To determine the effects of these peptides on mucin secretion, cells were preincubated with the peptides for 15 min prior to addition of secretagogues, and mucin secretion was then measured by ELISA.

*Antisense Oligonucleotides--* MARCKS antisense oligonucleotide and its corresponding control oligonucleotide were synthesized at Biognostik GmbH (Gottingen, Germany). NI-IBE cells were treated with 5 µM antisense or control oligonucleotide apically for 3 days (in the presence of 2 1.µg/ml lipofectin for the first 24 h). Cells were then incubated with secretagogues, and mucin secretion was measured by ELISA. Total RNA and protein were isolated from treated cells. MARCKS mRNA was assessed by Northern hybridization according to conventional procedures using human MARCKS cDNA as a probe. MARCKS protein level was determined by Western blot using purified anti-MARCKS IgG1 (clone 2F12) as the primary detection antibody.

*Transient Transfection-* The phosphorylation site domain (PSD) of MARCKS contains the PKC-dependent phosphorylation sites and the actin filament- binding site. To construct a PSD-deleted MARCKS eDNA, two fragments flanking the PSD sequence (coding for 25 amino acids) were generated by polymerase chain reaction and then ligated through the XhoI site that was attached to the 5'-ends of oligonueleotide primers designed for the polymerase chain reaction. The resultant mutant cDNA and the wild-type MARCKS cDNA were each inserted into a mammalian expression vector pcDNA4/TO (Invitrogen, Carlsbad, CA). Isolated recombinant constructs were confirmed by restriction digests and DNA sequencing.

HBE 1 is a papilloma virus-transformed human bronchial epithelial cell line capable of mucin secretion when cultured in air/liquid interface. Transfection of HBE1 cells was carried out using the Effectene transfection reagent (Qiagen, Valencia, CA) according to the manufacturer's instructions. Briefly, differentiated HBE1 cells grown in air/liquid interface were dissociated by trypsin1EDTA and reseeded in 12-well culture plates at 1 x 10⁵ cells/cm². After overnight incubation, cells were transfected with the wild-type MARCKS cDNA, the PSD-truncated MARCKS cDNA, or vector DNA. Cells were cultured for 48 h to allow gene expression and then exposed to secretagogues and mucin secretion measured by ELISA. All transfections were carried out in the presence of peDNA4/TO/lacZ plasmid (Invitrogen) (DNA ratio 6:1, total 1 µg DNA, ratio of DNA to Effectene reagent = 1:25) to monitor variations in transfection efficiency. Results showed no significant difference in β-galactosidase activities in cell lysates isolated from the transfected cells, indicating similar transfection efficiency among different DNA constructs (data not shown).

*Protein Phosphatase Activity Assay--* PP 1 and PP2A activities were measured using a protein phosphatase assay system (Life Technologies, Inc.) as known in the art with slight modification. Huang et al., Adv. Exp. Med. Biol. 396, 209-215 (1996). Briefly, NHBE cells were treated with 8-Br-cGMP or medium alone for 5 min. Cells were then scraped into a lysis buffer (50 mM Tris-HCl (pH 7.4), 0.1% (β-mecaptoethanol, 0.1 mM EDTA, 1 mM benzamidine, 10 µg/ml pepstatin A, 10 µg/ml leupeptin) and disrupted by sonication for 20 s at 4°C. Cell lysates were centrifuged and the supernatants saved for phosphatase activity assay. The assay was performed using ³²P-labeled phosphorylase A as a substrate. Released ³²Pi was counted by scintillation. The protein concentration of each sample was determined by the Bradford assay. PP2A activity was expressed as the sample total phosphatase activity minus the activity remaining in the presence of 1 nM okadaic acid. PP1 activity was expressed as the difference between the activities remaining in the presence of 1 nM and I p.M okadaic acid, respectively. Protein phosphatase activities were reported as nmol of Pi released per min/mg total protein.

*Cytotoxicity Assay--* All reagents used in treating NHBE cells were examined for cytotoxicity by measuring the total release of lactate dehydrogenase from the cells. The assay was carried out using the Promega Cytotox 96 Kit according to the manufacturer's instructions. All experiments were performed with reagents at non-cytotoxic concentrations.

*Statistical Analysis--* Data were analyzed for significance using one-way analysis of variance with Bonferroni post-test corrections. Differences between treatments were considered significant at *p* < 0.05.

*Isolation of PMNs from canine blood--* The steps involved in isolating PMN include collecting 10 ml ACD anticoagulated blood. Then layering 5 ml on 3.5 ml PMN isolation media while ensuring that the PMN isolation media (IM) was at room temperature (RI). Next, the blood was centrifuged at room temperature for 30', 550 X g at 1700 RPMs. The low lower white band was transferred into 15 ml conical centrifuge tube (CCFT). Next, 2V HESS with 10% fetal bovine serum (PBS) was added and centrifuged at room temperature for 10', 400 X g at 1400 RPMs. The pellet was then resuspended in 5 ml 1ESS with PBS. The cell suspension was added to 50 ml CCFT containing 20 ml of ice cold 0.88% NH4C1 and inverted two to three times. The resulting product was centrifuged for 10', 800 X g at 2000 RPMs, then aspirated and resuspended in 5 ml HBSS with FBS. The prep was examined by counting and cytospin and preferably for whole blood, the cell number should be between 10⁹-10" cells and for PMNs, cell number should be between 2-4 x 10⁷ cells. *See generally,* Wang et al., J. Immunol., "Neutrophil-induced changes in the biomechanical properties of endothelial cells: roles of ICAM-1 and reactive oxygen species," 648794 (2000).

*MPO Colorimetric Enzyme Assay--* Samples were assayed for MPO activity in 96 well round bottom microtiter plates using a sandwich ELISA kit (R & D Systems, Minneapolis, MN). Briefly, 20 microliters of sample is mixed with 180 microliters of substrate mixture containing 33 mM potassium phosphate, pH 6.0, 0.56% Triton X-100, 0.11 mM hydrogen peroxide, and 0.36 mM O-Diannisidine Dihydrochloride in an individual microtiter well. The final concentrations in the assay mixture are: 30 mM potassium phosphate, pH 6.0, 0.05% Triton X-100, 0.1 mM hydrogen peroxide, and 0.32 mM 0-Diannisidine Dihydrochloride. After mixing, the assay mixture was incubated at room temperature for 5 minutes, and MPO enzyme activity determined spectrophotometrically at 550 nanometers, Samples were assayed in duplicate.

The foregoing examples are illustrative of the present invention and are not to be construed as limiting thereof. The invention is defined by the following claims, with equivalents of the claims to be included therein.

## Claims

1. MANS peptide having the amino acid sequence of SEQ ID NO 1, or an active fragment thereof, for use in treating arthritis.

2. MANS peptide or an active fragment thereof according to Claim 1, wherein said arthritis is osteoarthritis or rheumatoid arthritis.

3. Use of MANS peptide having the amino acid sequence of SEQ ID NO 1, or an active fragment thereof, for the manufacture of a medicament for treating arthritis.

4. Use of MANS peptide or an active fragment thereof according to Claim 3, wherein said arthritis is osteoarthritis or rheumatoid arthritis.

5. An active fragment according to Claim 1 or Claim 2, or use of an active fragment according to Claims 3 or Claim 4, wherein said active fragment comprises at least six amino acids.

6. A MANS peptide or active fragment thereof for use in treating arthritis according to Claim 1 or Claim 2, for use in treating a mammalian subject.

7. A MANS peptide or active fragment thereof for use in treating arthritis according to Claim 6, wherein the mammalian subject is selected from the group consisting of: humans; canines; equines; and felines.

8. A MANS peptide or an active fragment thereof for use in treating arthritis according to Claim 1 or Claim 2, wherein said MANS peptide or an active fragment thereof is administered by topical administration, parenteral administration, rectal administration, nasal administration, or oral administration.

9. A MANS peptide or an active fragment thereof for use in treating arthritis according to Claim 1 or Claim 2, wherein said MANS peptide or an active fragment thereof is prepared for administration by topical administration, parenteral administration, rectal administration, nasal administration, or oral administration.

10. A MANS peptide or active fragment thereof for use in treating arthritis according to Claim 1 or Claim 2, wherein said treatment further comprises the use of a second molecule selected from the group consisting of: an antibiotic; an antiviral compound; an antiparasitic compound; an anti-inflammatory compound; and an immunosuppressant.

## Patentansprüche

1. MANS-Peptid mit einer Aminosäure-Sequenz gemäß SEQ ID Nr. 1, oder einem aktiven Fragment davon, zur Verwendung bei der Behandlung von Arthritis.

2. MANS-Peptid oder ein aktives Fragment davon, nach Anspruch 1, wobei die Arthritis Knochenarthritis oder rheumatische Arthritis ist.

3. Verwendung eines MANS-Peptids mit einer Aminosäure-Sequenz gemäß SEQ ID Nr. 1 oder einem aktiven Fragment davon, zur Herstellung eines Medikaments zur Behandlung von Arthritis.

4. Verwendung eines MANS-Peptids, oder eines aktiven Fragments davon, nach Anspruch 3, wobei die Arthritis Knochenarthritis oder rheumatische Arthritis ist.

5. Aktives Fragment nach Anspruch 1 oder 2, oder Verwendung eines aktiven Fragments nach Anspruch 3 oder 4, wobei das aktive Fragment mindestens sechs Aminosäuren umfasst.

6. MANS-Peptid, oder aktives Fragment davon, zur Verwendung bei der Behandlung von Arthritis nach Anspruch 1 oder 2, zur Verwendung bei der Behandlung eines Säugetiers.

7. MANS-Peptid, oder aktives Fragment davon, zur Verwendung bei der Behandlung von Arthritis nach Anspruch 6, wobei das Säugetier aus der Gruppe bestehend aus Menschen, Hundeartigen, Pferdeartigen und Katzenartigen ausgewählt ist.

8. MANS-Peptid, oder aktives Fragment davon, zur Verwendung bei der Behandlung von Arthritis nach Anspruch 1 oder 2, wobei das MANS-Peptid oder ein aktives Fragment davon verabreicht wird durch topische Verabreichung, parenterale Verabreichung, rektale Verabreichung, nasale Verabreichung oder orale Verabreichung.

9. MANS-Peptid, oder aktives Fragment davon, zur Verwendung bei der Behandlung von Arthritis nach Anspruch 1 oder 2, wobei das MANS-Peptid oder ein aktives Fragment davon aufbereitet ist für topische Verabreichung, parenterale Verabreichung, rektale Verabreichung, nasale Verabreichung oder orale Verabreichung.

10. MANS-Peptid oder aktives Fragment desselben zur Verwendung bei der Behandlung von Arthritis nach Anspruch 1 oder 2, wobei die Behandlung ferner die Verwendung eines zweiten Moleküls umfasst, das aus der Gruppe bestehend aus einem Antibiotikum, einer antiviralen Zusammensetzung, einer antiparasitären Zusammensetzung, einer entzündungshemmenden Zusammensetzung und einen Immunsuppressivum ausgewählt ist.

## Revendications

1. Peptide MANS ayant la séquence d'acides aminés de SEQ ID NO : 1, ou fragment actif de celui-ci, pour une utilisation dans le traitement de l'arthrite.

2. Peptide MANS ou fragment actif de celui-ci selon la revendication 1, où ladite arthrite est l'arthrose ou la polyarthrite rhumatoïde.

3. Utilisation du peptide MANS ayant la séquence d'acides aminés de SEQ ID NO : 1, ou d'un fragment actif de celui-ci, pour la fabrication d'un médicament destiné au traitement de l'arthrite.

4. Utilisation du peptide MANS ou d'un fragment actif de celui-ci selon la revendication 3, où ladite arthrite est l'arthrose ou la polyarthrite rhumatoïde.

5. Fragment actif selon la revendication 1 ou la revendication 2, ou utilisation d'un fragment actif selon la revendication 3 ou la revendication 4, où ledit fragment actif comprend au moins six acides aminés.

6. Peptide MANS ou fragment actif de celui-ci pour une utilisation dans le traitement de l'arthrite selon la revendication 1 ou la revendication 2, pour une utilisation dans le traitement d'un sujet mammalien.

7. Peptide MANS ou fragment actif de celui-ci pour une utilisation dans le traitement de l'arthrite selon la revendication 6, où le sujet mammalien est choisi dans le groupe constitué par : des êtres humains ; des canidés ; des équidés ; et des félidés.

8. Peptide MANS ou fragment actif de celui-ci pour une utilisation dans le traitement de l'arthrite selon la revendication 1 ou la revendication 2, où ledit peptide MANS ou un fragment actif de celui-ci est administré par administration topique, administration parentérale, administration rectale, administration nasale ou administration orale.

9. Peptide MANS ou fragment actif de celui-ci pour une utilisation dans le traitement de l'arthrite selon la revendication 1 ou la revendication 2, où ledit peptide MANS ou un fragment actif de celui-ci est préparé pour une administration par administration topique, administration parentérale, administration rectale, administration nasale ou administration orale.

10. Peptide MANS ou fragment actif de celui-ci pour une utilisation dans le traitement de l'arthrite selon la revendication 1 ou la revendication 2, où ledit traitement comprend en outre l'utilisation d'une seconde molécule choisie dans le groupe constitué par : un antibiotique ; un composé antiviral ; un composé antiparasitaire ; un composé anti-inflammatoire ; et un immunosuppresseur.
